(19) Europäisches Patentamt
European Patent Office
Office européen des brevets

(11) Numéro de publication : **0 313 472 B1**

# (12) FASCICULE DE BREVET EUROPEEN

(45) Date de publication du fascicule du brevet : **18.03.92 Bulletin 92/12**

(51) Int. Cl.$^5$ : **C07D 333/20**

(21) Numéro de dépôt : **88402666.7**

(22) Date de dépôt : **21.10.88**

## (54) Procédé de préparation de la N-(chloro-2 benzyl)(thiényl-2)-2 éthylamine et produits intermédiaires dans cette préparation.

Le dossier contient des informations techniques présentées postérieurement au dépôt de la demande et ne figurant pas dans le présent fascicule.

(30) Priorité : **22.10.87 FR 8714643**

(43) Date de publication de la demande : **26.04.89 Bulletin 89/17**

(45) Mention de la délivrance du brevet : **18.03.92 Bulletin 92/12**

(84) Etats contractants désignés : **AT BE CH DE ES FR GB GR IT LI LU NL SE**

(56) Documents cités :
**EP-A- 0 068 978**
**EP-A- 0 068 979**
**EP-A- 0 069 002**
**Régles de nomenclature pour la chimie, p. 298**
**Nomenclature of Org. Chem., p. 320**

(73) Titulaire : **SANOFI**
**40, Avenue George V**
**F-75008 Paris (FR)**

(72) Inventeur : **Radisson, Joel**
**10 avenue Winston Churchill**
**F-31100 Toulouse (FR)**

(74) Mandataire : **Varady, Peter et al**
**Cabinet Lavoix 2, Place d'Estienne d'Orves**
**F-75441 Paris Cedex 09 (FR)**

EP 0 313 472 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un procédé de préparation de la N-(chloro-2 benzyl) (thiényl)-2)-2 éthylamine à partir d'une imine.

Cette amine secondaire a été décrite pour la première fois dans le brevet FR-A-2 300 090 comme intermédiaire de synthèse du composé de formule :

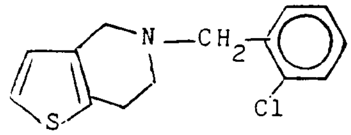

antiagrégant plaquettaire, dont la dénomination commune internationale est ticlopidine.

Le procédé de préparation de la N-(chloro-2 benzyl)(thiényl-2)-2 éthylamine de formule :

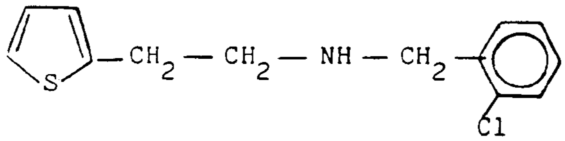

I

décrit dans le brevet FR-A-2 300 090, comprend la réaction de la chloro-2 benzylamine sur le benzène-sulfonate de (thiényl-2)-2 éthyle selon le schéma réactionnel :

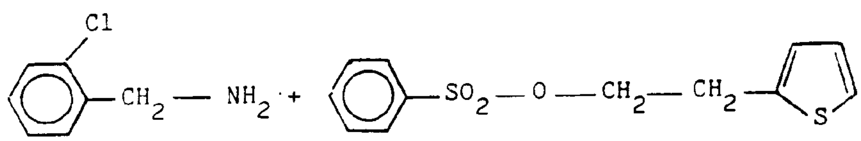

L'amine est disponible en quantités industrielles, mais le sulfonate de formule II doit être préparé à partir du (thiényl-2)-2 éthanol, obtenu par action de l'oxyde d'éthylène sur l'organolithien :

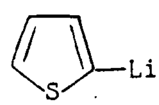

Bien que les rendements des réactions successives soient satisfaisants, l'ensemble du procédé est coûteux, d'autant plus qu'il implique de travailler, pour la première étape, en milieu rigoureusement anhydre.

EP-A-068 978 et -068 979 décrivent des procédés de préparation de thiényl-éthylamines N-aralkyl-subsitutées en 4 étapes, dont la dernière consiste en la réduction d'un azadiène (ène-imine) par un hydrure mixte de métal alcalin, notamment un borohydrure.

EP-A-069 002 concerne un procédé de préparation de la (thiényl-2 et -3)-2 éthylamine par hydrogénation catalytique du (thiényl-2 et -3)-2 nitroéthylène.

On a maintenant trouvé que le composé de formule I pouvait être préparé avec d'excellents rendements, par réduction d'une base de Schiff.

Un objet de l'invention est donc le procédé de préparation de la N-(chloro-2 benzyl) (thiényl-2)-2 éthylamine par réduction par voie chimique, électrochimique ou par hydrogénation catalytique d'une imine de formule

$$Ar^1\text{-}CH_2\text{-}N\text{=}CH\text{-}Ar^2 \qquad II$$

dans laquelle $Ar^1$ et $Ar^2$ sont différents et représentent chacun un groupe thényle-2 (c'est-à-dire thiényl-2 méthyle) ou chloro-2 phényle,
c'est-à-dire des composés de formule

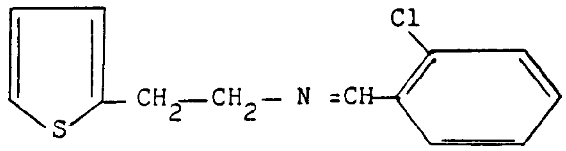

IIA

et

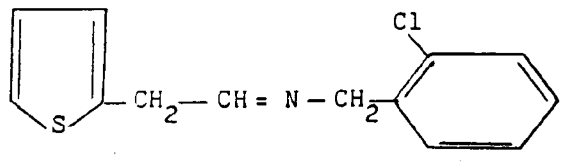

IIB

Les composés (II) sont des produits nouveaux et un autre objet de l'invention; ils peuvent être préparés avec d'excellents rendements par condensation d'un aldéhyde de formule $Ar^1CHO$ avec une amine primaire de formule $Ar^2CH_2NH_2$, dans lesquelles $Ar^1$ et $Ar^2$ ont les mêmes significations que dans la formule II.

La base de Schiff de formule IIA est obtenue par condensation de la chloro-2 benzaldéhyde sur la (thiényl-2)-2 éthylamine. Lorsqu'on opère à des températures comprises entre 20° et 80°C les rendements sont pratiquement quantitatifs.

La formation de la base de Schiff IIB est aussi rapide et pratiquement quantitative. Il suffit de mélanger les réactifs, le thiophène-2 acétaldéhyde devant être de préférence ajouté à la chloro-2 benzylamine, pure ou en solution.

Un des deux réactifs peut être en léger excès. On utilisera de préférence un excès exprimé en moles de 0 à 10% de chloro-2 benzylamine.

La réaction est conduite de préférence entre 0 et 40°C, de préférence entre 0 et + 5°C.

Les réactions de formation des bases de Schiff IIA et IIB peuvent être réalisées dans un solvant inerte, comme les alcools méthylique, éthylique, propylique ou butylique, les étheroxydes comme l'éther éthylique, isopropylique, le tétrahydrofuranne, le dioxanne, les hydrocarbures aromatiques comme le benzène, le toluène, le xylène. Il n'est pas nécessaire d'ajouter un agent déshydratant au milieu réactionnel.

Les bases de Schiff de formule IIA et IIB peuvent être réduites de façon classique par hydrogénation catalytique ou par réduction chimique.

– soit par l'hydrogène, en présence d'un catalyseur, comme le nickel ou le cobalt à l'état finement divisé, le platine, le palladium, le rhodium, le ruthénium;

– soit par un hydrure métallique, comme l'hydrure double d'aluminium et de lithium ou le borohydrure de sodium;

– soit par un métal suffisamment électropositif comme le sodium, le magnésium, le zinc, ou par tout autre agent réducteur comme le dithionite de sodium.

Les bases de Schiff peuvent également être réduites par voie électrochimique.

La réduction peut être effectuée après isolement des bases de Schiff de leur milieu de formation, ou simultanément à sa préparation, par exemple, en additionnant l'aldéhyde au milieu réactionnel contenant l'amine et l'agent réducteur.

Le produit de formule I est isolé par distillation ou par cristallisation de l'un de ses sels, notamment de son chlorhydrate.

Les exemples suivant illustrent l'invention.

EXEMPLE N° 1 :

N-(o-chlorobenzylidène) (thiényl-2)-2 éthylamine (composé de formule IIA)

127,5g (1 mole) de (thiényl-2)-2 éthylamine sont dissous dans 200 ml d'éther isopropylique. On ajoute en une fois 148 g (1,05 mole) de chloro-2 benzaldéhyde dissous dans 200 ml d'éther isopropylique. Après 30 mn, l'eau de réaction est décantée et la solution encore agitée 30 mn en présence de 20 g de sulfate de magnésium.

Le sulfate de magnésium est filtré, l'éther est évaporé et le résidu est distillé sous pression réduite.

On obtient 234,7 g de N-(o-chlorobenzylidène) (thiényl-2)-2 éthylamine distillant entre 140° et 150°C sous 0,4-0,5 mm de mercure (53 à 66 Pa). Rendement : 94%

Le produit est caractérisé par spectroscopie infra-rouge en film ($\gamma_{C=N}$ à 1640 cm $^{-1}$) et RMN du proton : 1

triplet (2H) à 3,25 ppm, 1 triplet (2H) à 3,95 ppm, protons aromatiques (7H) entre 6,8 et 8,2 ppm, 1 singulet à 8,65 ppm (1H) (solvant : $CDCl_3$; référence interne : tétraméthylsilane).

EXEMPLE 2 :

Chlorhydrate de N-(chloro-2 benzyl) (thiényl-2)-2 éthylamine

A une solution de 28,1 g (0,20 mole) de chloro-2 benzaldéhyde dans 140 ml d'éthanol, on ajoute 26,0 g (0,20 ml) de (thiényl-2)-2 éthylamine et chauffe 40 mn à 55°C. On ajoute ensuite, à 55°C, en 10 mn, 15,1 g (0,40 mole) de borohydrure de sodium et agite encore 5mn. La solution est ramenée à la température ambiante. Elle est additionnée de 200 g de glace et de 60 ml d'acide chlorhydrique (d:1,18). Le produit qui a cristallisé est filtré, lavé à l'eau et à l'alcool et séché à 60°C à poids constant.

On obtient ainsi 52,2 g de chlorhydrate de N-(chloro-2 benzyl) (thiényl-2)-2 éthylamine.

P.F. = 148°C. RMN conforme. Rendement : 90,6%

EXEMPLE 3 :

Chlorhydrate de N-(chloro-2 benzyl) (thiényl-2)-2 éthylamine

A une solution de 26,0 g (0,20 mole) de (thiényl-2)-2 éthylamine dans 150 ml de méthanol, on ajoute 30,9 g (0,22 mole) de chloro-2 benzaldéhyde. On agite 30 mn à 25°C, puis 30 mn à reflux. A la solution ramenée à 10°C, on ajoute 8,3g (0,22 mole) de $NaBH_4$ et agite 30 mn à température ambiante.

Après évaporation du méthanol, le résidu est repris par 100 g de glace et 60 ml d'acide chlorhydrique concentré. Le produit aui a cristallisé est filtré, lavé à l'eau, à l'éther et à l'acétone et séché à 50°C à poids constant.

On obtient ainsi 54,75 g de chlorhydrate de N-(chloro-2 benzyl) (thiényl-2)-2 éthylamine.

PF = 148°C. Rendement : 95,0 %

EXEMPLE 4 :

Chlorhydrate de N-(chloro-2 benzyl) (thiényl-2)-2 éthylamine

En opérant comme dans l'exemple 3, mais avec 5,9 g (0,15 mole) de borohydrure de sodium, on obtient 54,5 g de chlorhydrate de N-(chloro-2 benzyl) (thiényl-2)-2 éthylamine.

Rendement : 94,6%

EXEMPLE 5 :

Chlorhydrate de N-(chloro-2 benzyl) (thiényl-2)-2 éthylamine

A une solution de 26,0 g (0,20 mole de (thiényl-2)-2 éthylamine dans 50 ml d'éther isopropylique on ajoute 28,1 g (0,20 mole) de chloro-2 benzaldéhyde en solution dans 50 ml d'éther isoprpylique. Après 10 mn de chauffe à reflux, l'eau de réaction est décantée, la phase éthérée est séchée sur Mg $SO_4$ et filtrée. On ajoute ensuite en 30 mn une solution de 7,6 g (0,2 mole) de $LialH_4$ dans 100 ml d'éther anhydre et chauffe encore 1 h à reflux. On ajoute ensuite 50 ml d'acétate d'éthyle, 13 ml d'eau, 3 ml de NaOH 15% et encore 40 ml d'eau.

Les produits minéraux sont filtrés et rincés par 200 ml d'éther isopropylique. Le filtrat est additionné de 100 ml d'acide chlorhydrique 4N. Après 1/2 h d'agitation, le produit qui a cristallisé est filtré, rincé à l'eau et à l'éther, et séché à 60°C à poids constant.

On obtient 50,7 g de chlorhydrate de N-(chloro-2 benzyl) (thiényl-2)-2 éthylamine.

PF = 147°C. Rendement : 88%

EXEMPLE 6 :

Chlorhydrate de N-(chloro-2 benzyl) (thiényl-2)-2 éthylamine

25 g (0,196 mole) de (thiényl-2)-2 éthylamine sont mélangés à 30,3 g (0,215 mole) de chloro-2 benzaldéhyde dans 100 ml de toluène. Après 15 mn, l'eau de réaction est décantée et le toluène est chassé sous pression réduite. Le résidu (constitué essentiellement par la base de Shiff (IIA) est repris par 200 ml d'éthanol et est hydrogéné en présence de 5g de nickel de Raney, à 50°C, sous une pression de 20 bars (2MPa) . Dès que la quantité stoéchiométrique en hydrogène est absorbée, l'hydrogène est purgé, le nickel de Raney est filtré et l'alcool évaporé. Le concentrat est agité avec 200 ml d'éther isopropylique, 100 g de glace et 60 ml d'acide chlorhydrique concentré. Le produit qui a cristallisé est filtré, lavé à l'eau, à l'éther et à l'acétone, puis séché à 60°C à poids constant.

On obtient 52,3 g de chlorhydrate de N- (chloro-2 benzyl) (thiényl-2)-2 éthylamine.
P.F. = 148°C. Rendement : 92,9 %

EXEMPLE 7 :

Chlorhydrate de N-(chloro-2 benzyl) (thiényl-2)-2 éthylamine

12,7 g (0,10 mole) de (thiényl-2)-2 éthylamine sont mélangés à 14,0 g (0,10 mole) de chloro-2 benzaldéhyde, dans 100 ml de toluène. Après décantation de l'eau de réaction et évaporation du toluène, la base de Schiff obtenue ci-dessus est hydrogénée dans 200 ml d'éthanol, en présence de 5 g de charbon palladié, à 40°C sous une pression de 10 bars (1MPa ). Dès que la quantié stoéchiométrique en hydrogène est absorbée, l'hydrogène est immédiatement purgé, le catalyseur filtré. Après le traitement final comme dans l'exemple 6, on obtient 24,5 g de chlorhydrate de N-(chloro-2 benzyl) (thiényl-2)-2 éthylamine.

PF = 146°C. Rendement : 85%

EXEMPLE 8 :

Chlorhydrate de N-(chloro-2 benzyl) (thiényl-2)-2 éthylamine

0,1 mole de base de Schiff brute (IIA) préparée comme dans l'exemple 6 sont chauffés à reflux dans 100 ml de méthanol anhydre en présence de 5 g de magnésium sec; après 1 h, on dilue par 50 ml de méthanol supplémentaire, ajoute 3 g de magnésium et chauffe encore 2 h à reflux.

Le méthanol est alors évaporé, le résidu repris dans 50 ml d'acide chlorhydrique concentré et 100 g de glace. Après une demi-heure d'agitation, le produit est filtré, lavé à l'eau, à l'éther isopropylique et à l'acétone, séché à 60°C à poids constant.

On obtient 27,7 g de chlorhydrate de N-(chloro-2 benzyl) (thiényl-2)-2 éthylamine.

PF = 147°C. Rendement : 96,2%

EXEMPLE 9

A 0,1 mole de base de Schiff (IIA) préparée selon l'exemple 6, dissous dans une solution de 25 ml de lessive de soude (400 g/l) dans 100 ml d'éthanol, on ajoute par petites fractions 13 g de poudre de zinc et agite pendant 18 h. Le mélange réactionnel est en suite filtré, l'alcool évaporé. Le résidu d'évaporation est repris par 100 g de glace et 50 ml d'acide chlorhydrique concentré. Le produit qui a précipité est filtré, lavé à l'eau, à l'éther isopropylique et à l'acétone et est séché à 60°C à poids constant.

On obtient 22,7 g de chlorhydrate de N-(chloro-2 benzyl) (thiényl-2)-2 éthylamine.

P.F. = 147°C. Rendement : 78,8 %

EXEMPLE 10 :

34,8 g de dithionite de sodium (0,20 mole) sont suspendus sous azote dans un mélange de 150 ml d'éthanol et de 50 ml d'eau, puis on ajoute 0,1 mole de base de Schiff (IIA) obtenue selon l'exemple 6. Après 18 h d'agitation à température ambiante et 1 h de chauffage à reflux, la suspension est filtrée, l'insoluble lavé par 100 ml d'éthanol et le filtrat évaporé à sec. Le résidu est repris par 100 g de glace, 60 ml d'acide chlorhydrique concentré et 200 ml d'éther isopropylique. Le produit qui a cristallisé est filtré, lavé à l'eau, à l'éther, à l'acétone et séché à 60°C à poids constant.

On obtient 24,8 g de chlorhydrate de N-(chloro-2 benzyl) (thiényl-2)-2 éthylamine.

P.F. = 148°C. Rendement : 86.0%

EXEMPLE 11 :

14,5 g (0,058 mole) de base Schiff (IIA) préparé dans le toluène comme dans l'exemple 6 sont dissous dans une solution d'acétate de potassium (30 g) dans 160 ml d'éthanol et 40 ml d'eau. La solution est placée dans le compartiment cathodique d'un électrolyseur, tandis que le compartiment anodique contient une solution de 100 g d'acétate de potassium dans 160 ml d'éthanol à 20% d'eau. La cathode est une nappe de mercure, l'anode est en graphite. L'électro-lyse est effectuée à 20°C, avec un courant de 2 ampères pendant 3 h. Le catholyte est ensuite dilué par 100 ml d'eau, extrait par deux fois 200 ml d'éther isoporpylique puis la phase éthérée est agitée avec 100 ml d'acide chlorhydrique 6N. Le produit qui a précipité est filtré, lavé à l'eau, à l'éther et à l'acétone et est séché à 60°C à poids constant.

On obtient 14,35 g de chlorhydrate de N-(chloro-2 benzyl) (thiényl-2)-2 éthylamine.

P.F. = 147°C. Rendement : 85,6%

EXEMPLE 12 :

17,8 g (0,10 mole) de chlorhydrate de chloro-2 bensylamine et 28,2 g (0,2 mole) de chloro-2 benzylamine sont dissous dans 150 ml de méthanol. On ajoute 4,4 g (0,07 mole) de cyanoborohydrure de sodium, puis en 30 mn, 12,6 g (0,10 mole) de thiophène-2 acétaldéhyde entre 10°C et 20°C et agite pendant 30 heures à 25°C. Le mélange réactionnel est alors coulé sur 200 g de glace et 200 ml d'acide chlorhydrique concentré. Après 15 mn d'agitation, on ajoute 200 ml d'éther isopropylique et agite encore 15 mn. Le chlorhydrate de N-(chloro-2 benzyl) (thiényl-2)-2 éthylamine qui a cristallisé est filtré, lavé à l'eau, à l'éther et à l'acétone et est séché à poids constant. On obtient 21,9 g de produit attendu.

Rendement : 76% (par rapport au thiophène acétaldéhyde).

EXEMPLE 13 :

A 28,2g (0,2 mole) de chloro-2 benzylamine refroidi à 0°C, on ajoute en 30 mn 22,7 g (0,18 mole) de thiophène-2 acétaldéhyde, à température comprise entre 0 et 10°C. On obtient ainsi la N-(thiényl-2)-2 éthylidène chloro-2 benzylamine (formule IIB) qui est caractérisée par spectroscopie infrarouge ($\gamma_{C=N}$ à 1640 $cm^{-1}$) et spectroscopie RMN du proton : 1 méthylène singulet à 3,8 ppm, 1 méthylène doublet à 3,75 ppm, protons aromatiques entre 6,7 et 7,4 ppm et méthine à 7,75 ppm (solvant : $CDCl_3$; référence interne : tétraméthylsilane).

La base de Schiff (IIB) est dissoute dans 200 ml de méthanol puis est hydrogénée en présence de 5 g de nickel de Raney à 20°C, sous une pression d'hydrogène de 5 bars (500 KPa).

Le nickel de Raney est filtré, le méthanol évaporé, le résidu repris par 200 ml d'éther isopropylique et agité 30 mn en présence de 200 ml d'acide chlorhydrique 6N.

Le chlorhydrate de N-(chloro-2 benzyl) (thiényl-2)-2 éthylamine qui a cristallisé est filtré, lavé à l'eau, à l'éther et à l'acétone et est séché à poids constant. On obtient 45,6 g de produit attendu.

Rendement : 88%.

## Revendications

**Revendications pour les Etats contractants suivants : DE, GB, FR, IT, NL, SE, CH, LI, BE, AT, LU**

1. Procédé de préparation du produit de formule

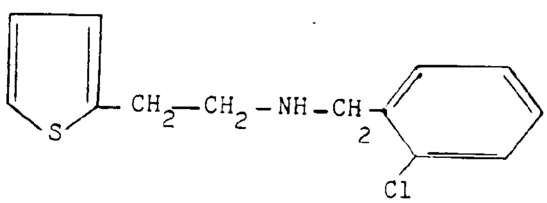 I

caractérisé en ce que l'on réduit par voie chimique, électrochimique ou par hydrogénation catalytique une imine de formule

$$Ar^1\text{-}CH_2\text{-}N{=}CH\text{-}Ar^2 \qquad II$$

dans laquelle $Ar^1$ et $Ar^2$ sont différents et représentent chacun un groupe thényle-2 ou chloro-2 phényle.

2. Procédé selon la revendication 1, caractérisé en ce que l'imine (II) est réduite par l'hydrogène en présence d'un catalyseur.

3. Procédé selon la revendication 1, caractérisé en ce que l'imine (II) est réduite par action d'un hydrure métallique.

4. Procédé selon la revendication 1, caractérisé en ce que l'imine (II) est réduite par le dithionite de sodium.

5. Procédé selon la revendication 1, caractérisé en ce que l'imine (II) est réduite par un métal électropositif.

6. Procédé selon la revendication 1, caractérisé en ce que la réduction de l'imine (II) est effectuée simultanément lors de sa préparation à partir d'un aldéhyde $Ar^1CHO$ sur une amine $Ar^2CH_2NH_2$, dans lesquelles $Ar^1$ et $Ar^2$ ont la même signification que dans la formule I.

7. Procédé selon la revendication 1, caractérisé en ce que l'imine (II) est réduite par voie électrochimique.

8. Produit de formule

$$Ar^1\text{-}CH_2\text{-}N\text{=}CH\text{-}Ar^2 \quad II$$

dans laquelle $Ar^1$ et $Ar^2$, différents, représentent chacun un groupe thényle-2 ou chloro-2 phényle.

9. Procédé de préparation du produit de formule II selon la revendication 7, caractérisé en ce que l'on condense un aldéhyde $Ar^1CHO$ avec une amine $Ar^2CH_2NH_2$, dans lesquelles $Ar^1$ et $Ar^2$ ont la même signification que dans la formule II.

**Revendications pour les Etats contractants suivants: ES, GR**

1. Procédé de préparation du produit de formule

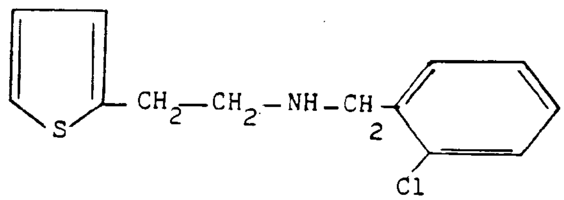

I

caractérisé en ce que l'on réduit par voie chimique, électrochimique ou par hydrogénation catalytique une imine de formule

$$Ar^1\text{-}CH_2\text{-}N\text{=}CH\text{-}Ar^2 \quad II$$

dans laquelle $Ar^1$ et $Ar^2$ sont différents et représentent chacun un groupe thényle-2 ou chloro-2 phényle.

2. Procédé selon la revendication 1, caractérisé en ce que l'imine (II) est réduite par l'hydrogène en présence d'un catalyseur.

3. Procédé selon la revendication 1, caractérisé en ce que l'imine (II) est réduite par action d'un hydrure métallique.

4. Procédé selon la revendication 1, caractérisé en ce que l'imine (II) est réduite par le dithionite de sodium.

5. Procédé selon la revendication 1, caractérisé en ce que l'imine (II) est réduite par un métal électropositif.

6. Procédé selon la revendication 1, caractérisé en ce que la réduction de l'imine (II) est effectuée simultanément lors de sa préparation à partir d'un aldéhyde $Ar^1CHO$ sur une amine $Ar^2CH_2NH_2$, dans lesquelles $Ar^1$ et $Ar^2$ ont la même signification que dans la formule I.

7. Procédé selon la revendication 1, caractérisé en ce que l'imine (II) est réduite par voie électrochimique.

8. Procédé de préparation du produit de formule II

$$Ar^1\text{-}CH_2\text{-}N\text{=}CH\text{-}Ar^2$$

dans laquelle $Ar^1$ et $Ar^2$, différents, représentent chacun un groupe thényle-2 ou chloro-2 phényle, caractérisé en ce que l'on condense un aldéhyde $Ar^1CHO$ avec une amine $Ar^2CH_2NH_2$, dans lesquelles $Ar^1$ et $Ar^2$ ont la même signification que dans la formule II.

## Claims

**Claims for the following Contracting States : DE, GB, FR, IT, NL, SE, CH, LI, BE, AT, LU**

1. A process for preparing the product of the formula:

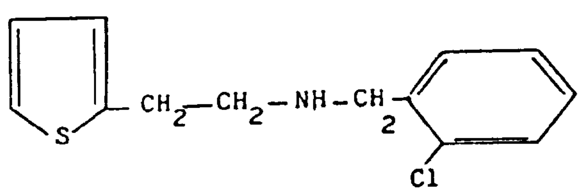

I

characterised in that an imine of formula II

$$Ar^1\text{-}CH_2\text{-}N\text{=}CH\text{-}Ar^2 \quad II$$

in which $Ar^1$ and $Ar^2$ are different and each represents a 2-thenyl group or 2-chlorophenyl group, is reduced by chemical or electromechanical means or by catalytic hydrogenation.

2. A process according to Claim 1, characterised in that the imine (II) is reduced by hydrogen in the presence

of a catalyst.

3. A process according to Claim 1, characterised in that the imine (II) is reduced by means of a metal hydride.

4. A process according to Claim 1, characterised in that the imine (II) is reduced by sodium dithionite.

5. A process according to Claim 1, characterised in that the imine (II) is reduced by an electropositive metal.

6. A process according to Claim 1, characterised in that the reduction of the imine (II) is carried out simultaneously with its preparation from an aldehyde $Ar^1CHO$ and an amine $Ar^2CH_2NH_2$, in which $Ar^1$ and $Ar^2$ have the same meaning as in formula I.

7. A process according to Claim 1, characterised in that the imine (II) is reduced by electrochemical means.

8. A product of the formula

$$Ar^1\text{-}CH_2\text{-}N{=}CH\text{-}Ar^2 \qquad II$$

in which $Ar^1$ and $Ar^2$ are different and each represents a 2-thenyl group or a 2-chlorophenyl group.

9. A process for preparing the product of the formula II according to Claim 7, characterised in that an aldehyde $Ar^1CHO$ is condensed with an amine $Ar^2CH_2NH_2$, in which $Ar^1$ and $Ar^2$ have the same meaning as in formula II.

**Claims for the followings Contracting States : ES, GR**

1. A process for preparing the product of the formula:

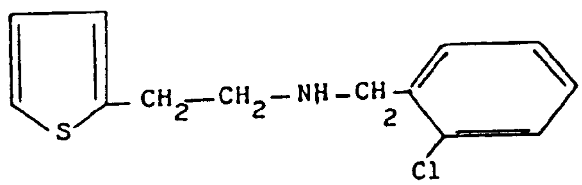

I

characterised in that an imine of formula II

$$Ar^1\text{-}CH_2\text{-}N{=}CH\text{-}Ar^2 \qquad II$$

in which $Ar^1$ and $Ar^2$ are different and each represents a 2-thenyl group or 2-chlorophenyl group, is reduced by chemical or electromechanical means or by catalytic hydrogenation.

2. A process according to Claim 1, characterised in that the imine (II) is reduced by hydrogen in the presence of a catalyst.

3. A process according to Claim 1, characterised in that the imine (II) is reduced by means of a metal hydride.

4. A process according to Claim 1, characterised in that the imine (II) is reduced by sodium dithionite.

5. A process according to Claim 1, characterised in that the imine (II) is reduced by an electropositive metal.

6. A process according to Claim 1, characterised in that the reduction of the imine (II) is carried out simultaneously with its preparation from an aldehyde $Ar^1CHO$ and an amine $Ar^2CH_2NH_2$, in which $Ar^1$ and $Ar^2$ have the same meaning as in formula I.

7. A process according to Claim 1, characterised in that the imine (II) is reduced by electrochemical means.

8. A processs for preparing the product of formula II

$$Ar^1\text{-}CH_2\text{-}N{=}CH\text{-}Ar^2$$

in which $Ar^1$ and $Ar^2$ are different and each represents a 2-thenyl group or a 2-chlorophenyl group, characterised in that an aldehyde $Ar^1CHO$ is condensed with an amine $Ar^2CH_2NH_2$, in which $Ar^1$ and $Ar^2$ have the same meaning as in formula II.

## Patentansprüche

**Patentansprüche für folgende vertragsstaaten : DE, GB, FR, IT, NL, SE, CH, LI, BE, AT, LU**

1. Verfahren zur Herstellung des Produkts mit der Formel

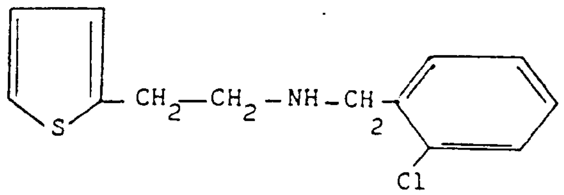

I

dadurch **gekennzeichnet** , daß man auf chemischem oder elektrochemischem Weg oder durch katalytische Hydrierung ein Imin mit der Formel

$Ar^1-CH_2-N=CH-Ar^2$ II

reduziert, worin $Ar^1$ und $Ar^2$ verschieden sind und jedes eine 2-Thienylgruppe oder eine 2-Chlorphenylgruppe darstellt.

2. Verfahren nach Anspruch 1, dadurch gekennzeichnet, daß das Imin (II) mit Wasserstoff in Gegenwart eines Katalysators reduziert wird.

3. Verfahren nach Anspruch 1, dadurch gekennzeichnet, daß das Imin (II) durch Einwirkung eines Metallhydrids reduziert wird.

4. Verfahren nach Anspruch 1, dadurch gekennzeichnet, daß das Imin (II) mit Natriumdithionit reduziert wird.

5. Verfahren nach Anspruch 1, dadurch gekennzeichnet, daß das Imin (II) mit einem elektropositiven Metall reduziert wird.

6. Verfahren nach Anspruch 1, dadurch gekennzeichnet, daß die Reduktion des Imins (II) gleichzeitig während seiner Herstellung durch Einwirkung eines Aldehyds $Ar^1CHO$ auf ein Amin $Ar^2CH_2NH_2$ durchgeführt wird, wobei $Ar^1$ und $Ar^2$ die gleiche Bedeutung wie in Formel I haben.

7. Verfahren nach Anspruch 1, dadurch gekennzeichnet, daß das Imin (II) auf elektrochemischem Wege reduziert wird.

8. Verfahren zur Herstellung des Produkts mit der Formel II

$Ar^1-CH_2-N=CH-Ar^2$ II

worin $Ar^1$ und $Ar^2$ verschieden sind und jedes eine 2-Thienylgruppe oder eine 2-Chlorphenylgruppe darstellt.

9. Verfahren zur Herstellung des Produkts mit der Formel II nach Anspruch 7, dadurch gekennzeichnet, daß man einen Aldehyd $Ar^1CHO$ mit einem Amin $Ar^2CH_2NH_2$ kondensiert, worin $Ar^1$ und $Ar^2$ die gleiche Bedeutung haben wie in Formel II.

**Patentansprüche für folgende vertragsstaaten : ES, GR**

1. Verfahren zur Herstellung des Produkts mit der Formel

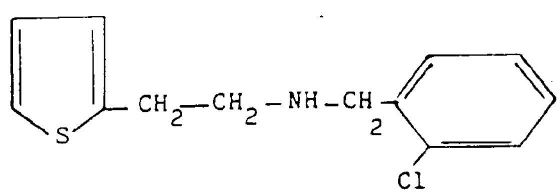

I

dadurch **gekennzeichnet** , daß man auf chemischem oder elektrochemischem Weg oder durch katalytische Hydrierung ein Imin mit der Formel

$Ar^1-CH_2-N=CH-Ar^2$ II

reduziert, worin $Ar^1$ und $Ar^2$ verschieden sind und jedes eine 2-Thienylgruppe oder eine 2-Chlorphenylgruppe darstellt.

2. Verfahren nach Anspruch 1, dadurch gekennzeichnet, daß das Imin (II) mit Wasserstoff in Gegenwart eines Katalysators reduziert wird.

3. Verfahren nach Anspruch 1, dadurch gekennzeichnet, daß das Imin (II) durch Einwirkung eines Metallhydrids reduziert wird.

4. Verfahren nach Anspruch 1, dadurch gekennzeichnet, daß das Imin (II) mit Natriumdithionit reduziert wird.

5. Verfahren nach Anspruch 1, dadurch gekennzeichnet, daß das Imin (II) mit einem elektropositiven Metall reduziert wird.

6. Verfahren nach Anspruch 1, dadurch gekennzeichnet, daß die Reduktion des Imins (II) gleichzeitig während seiner Herstellung durch Einwirkung eines Aldehyds $Ar^1CHO$ auf ein Amin $Ar^2CH_2NH_2$ durchgeführt wird, wobei $Ar^1$ und $Ar^2$ die gleiche Bedeutung wie in Formel I haben.

7. Verfahren nach Anspruch 1, dadurch gekennzeichnet, daß das Imin (II) auf elektrochemischem Wege reduziert wird.

8. Verfahren zur Herstellung des Produkts mit der Formel II

$$Ar^1\text{-}CH_2\text{-}N{=}CH\text{-}Ar^2 \qquad II$$

worin $Ar^1$ und $Ar^2$ verschieden sind und jedes eine 2-Thienylgruppe oder eine 2-Chlorphenylgruppe darstellt, dadurch gekennzeichnet, daß man einen Aldehyd $Ar^1CHO$ mit einem Amin $Ar^2CH_2NH_2$ kondensiert, worin $Ar^1$ und $Ar^2$ die gleiche Bedeutung haben wie in Formel II.